(19) Europäisches Patentamt
European Patent Office
Office européen des brevets

(11) EP 2 535 056 A2

(12) **EUROPEAN PATENT APPLICATION**
published in accordance with Art. 153(4) EPC

(43) Date of publication:
**19.12.2012 Bulletin 2012/51**

(21) Application number: **11742504.1**

(22) Date of filing: **11.02.2011**

(51) Int Cl.:
***A61K 36/236*** *(2006.01)* ***A61P 31/12*** *(2006.01)*

(86) International application number:
**PCT/KR2011/000940**

(87) International publication number:
**WO 2011/099812 (18.08.2011 Gazette 2011/33)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.02.2010 KR 20100013543**

(71) Applicant: **Korea Research Institute of Bioscience and Biotechnology**
**Yuseong-gu, Daejeon 305-333 (KR)**

(72) Inventors:
- **LEE, Woo Song**
  **Jeongeup-si**
  **Jeollabukdo 580-185 (KR)**
- **RHO, Mun Chual**
  **Jeongeup-si**
  **Jeollabukdo 580-185 (KR)**
- **RYU, Young Bae**
  **Gwangju 506-773 (KR)**
- **PARK, Su Jin**
  **Gwangju 502-775 (KR)**
- **CHANG, Jong Sun**
  **Jeongeup-si**
  **Jeollabukdo 580-185 (KR)**
- **KWON, Hyung Jun**
  **Daejeon 306-826 (KR)**
- **KIM, Ha Hyun**
  **Gwangju 500-796 (KR)**
- **JEONG, Hyung Jae**
  **Jinju-si**
  **Gyeongsangnam-do 660-761 (KR)**

(74) Representative: **Browne, Robin Forsythe et al**
**Hepworth Browne**
**Pearl Chambers**
**22 East Parade**
**Leeds LS1 5BY (GB)**

(54) **COMPOSITION FOR PREVENTING OR TREATING ROTAVIRUS INFECTION CONTAINING LICORICE EXTRACT**

(57) The present invention relates to a composition for preventing or treating rotavirus infection, comprising a licorice extract, fractions thereof or compounds separated therefrom. The licorice extract, fractions thereof or compounds separated therefrom have anti-rotavirus effects and exhibit not only virucidal effects against various rotaviruses, but also the effect of inhibiting the cytopathic effect of rotaviruses. Thus, the composition can be effectively used for the prevention or treatment of rotavirus infection.

EP 2 535 056 A2

## Description

### Technical Field

The present invention relates to a pharmaceutical composition for the prevention or treatment of rotavirus infection, which comprises, as an active ingredient, one or more of a licorice extract, fractions thereof or polyphenolic compounds separated therefrom, and to a method of using the pharmaceutical composition to prevent or treat rotavirus infection. Moreover, the present invention relates to a food composition and virucidal agent for the prevention or amelioration of rotavirus infection, which comprises, as an active ingredient, one or more of a licorice extract, fractions thereof or polyphenolic compounds separated therefrom. In addition, the present invention relates to the use of one or more of a licorice extract, fractions thereof or polyphenolic compounds separated therefrom in the preparation of a medicament for treating rotavirus infection.

### Background Art

Rotavirus has been recognized as one of the most common causes of severe diarrhea in young animals (cattle, pigs and goats) and human infants in the world. It is known that rotavirus can cause, in addition to severe diarrhea, diseases such as encephalitis, otitis media, necrotic colitis, liver abscess or intussusception (Ester, M. K. Rotaviruses and Their Replication in Fields Virology. pp. 1747-1785, 2001). Rotavirus belongs to the family *Reoviridae* and consists of viral particles having a diameter of about 70 nm. Its genome is composed of 11 segments of double-stranded RNA and is surrounded by three capsid layers.

Of these three capsid layers, the outermost layer consists of VP4 and VP7 proteins. The VP4 protein is cleaved into VP5 and VP8 by host protease, and thus the serotype determined by VP4 is referred to as P type. Also, the VP7 protein is glycosylated, and thus the serotype determined by VP7 is referred to as G type (Ciarlet, M. and Ester, M. K. Rotaviruses: basic biology, epidemiology and methodologies in encyclopedia of environmental microbiology. pp. 2753-2773, 2002). The VP4 and VP7 protein play an important role in the defense of the host by inducing neutralizing antibodies.

Rotaviruses are divided, according to the antigenic specificity of the intermediate capsid protein VP6, into seven serotypes (A-G). Among them, serotype A rotavirus most often attacks humans and animals and is known as a very important infectious disease that causes enteritis in 130,000,000 people annually worldwide and results in the death of 873,000 people each year.

In the case of serotype A rotavirus, G and P types specific to the respective hosts have been reported. In humans, a combination of G1-G3 and G9 and a combination of P[4] and P[8] are predominant (Kapikian et al., 2001). In pigs, a combination of G3-G5 and G11 and a combination of P[6] and P[7] are most predominant (Gouvea and Timenetsky, 1994). In cattle, a combination of G6, G8 and G10 and a combination of P[1], P[5] and P[11] predominantly appear (Alfieri et al., 2004). However, similar to influenza viruses, combinations of human and animal serotype A rotaviruses infect humans in nations in which humans come into frequent contact with animals, thus causing recombinant viruses which infect humans (Jain et al., 2001). In addition, in Brazil or the USA, G9 rotaviruses which emerge mainly in humans also emerged in pigs (Paul et al., 1988). This detection of animal rotaviruses in humans means that the cause of the infection is zoonosis. Moreover, rotaviruses can spread between different species of animals.

Methods which can be used to treat such viral diseases include the inhibition of virus adsorption to epithelial cells, the inhibition of virus penetration into cells, the inhibition of transcription and replication of virus genes, the inhibition of synthesis of virus proteins, and the inhibition of release from cells. These methods target antiviral actions, but a clear target for rotaviruses has not yet been found.

To inhibit rotavirus infection, many vaccines have been developed. In particular, for humans, animal-human recombinant virus vaccines made by recombination of human and animal rotaviruses have been produced and marketed. Typically, RotaTeq (Merck), a vaccine based on the bovine rotavirus strain WC3 and comprising genes corresponding to VP4 of rotavirus, was approved by the US FDA in 2006 and is being marketed. However, this recombinant virus vaccine was reported to cause adverse effects such as intussusception and has a significant shortcoming in that it cannot prevent various rotavirus serotypes.

To overcome this shortcoming, studies on natural materials capable of inhibiting rotavirus have been conducted. Typically, Takahashi et al. reported that a hot-water extract of stevia has anti-rotavirus effects (Takahashi, K et al., Antiviral Res. 49, 15, 2001).

## Disclosure

### Technical Problem

Accordingly, the present inventors have found that a licorice extract, fractions thereof or compounds separated therefrom have the activity of inhibiting rotavirus infection and exhibit not only virucidal effects against rotavirus, but also the effect of inhibiting the cytopathic effect of rotavirus, thereby completing the present invention.
The present invention is based on this finding.

### Technical Solution

The present invention provides a pharmaceutical composition for the prevention or treatment of rotavirus infection, comprising one or more of a licorice extract, fractions thereof or compounds represented by formulae 1 to 5 as an active ingredient.
The present invention also provides a food composition for the prevention or amelioration of rotavirus infection, comprising one or more of a licorice extract, fractions thereof or compounds represented by formulae 1 to 5 as an active ingredient.
The present invention also provides a virucidal agent against rotavirus comprising one or more of a licorice extract, fractions thereof or compounds represented by formulae 1 to 5 as an active ingredient.
The present invention also provides a method of preventing or treating rotavirus infection disease, comprising administering a pharmaceutical composition for preventing or treating rotavirus infection to a subject having or being at risk of developing rotavirus infection disease, wherein the composition comprises one or more of a licorice extract, fractions thereof or compounds represented by formulae 1 to 5 as an active ingredient.
The present invention also provides the use of one or more of a licorice extract, fractions thereof and compounds represented by formulae 1 to 5 in the preparation of a medicament for treating rotavirus infection disease.

### Advantageous Effects

The inventive compositions comprising a licorice extract, fractions thereof or polyphenolic compounds separated therefrom exhibit the effect of inhibiting the activity of rotavirus and have not only virucidal effects against a variety of rotaviruses, but also the effect of inhibiting the cytopathic effect of rotaviruses. Thus, the compositions of the present invention can be effectively used for the prevention or treatment of rotavirus infection disease.

### Best Mode

In one aspect, the present invention provides a pharmaceutical composition for the prevention or treatment of rotavirus infection, comprising one or more of a licorice extract, fractions thereof or compounds represented by the following formulae 1 to 5 as an active ingredient:

[Formula 1]

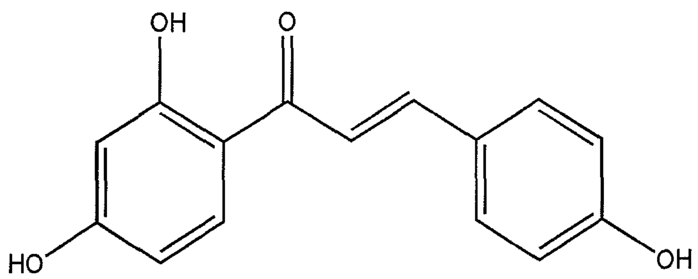

[Formula 2]

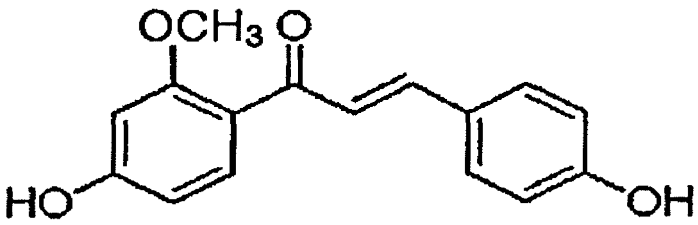

[Formula 3]

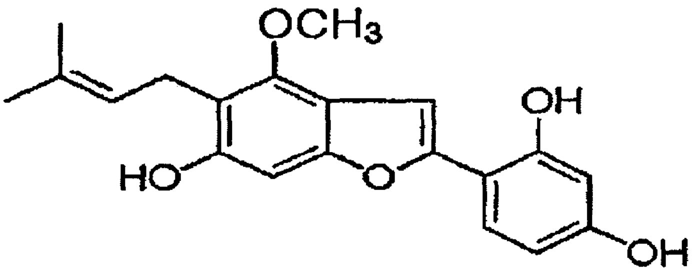

[Formula 4]

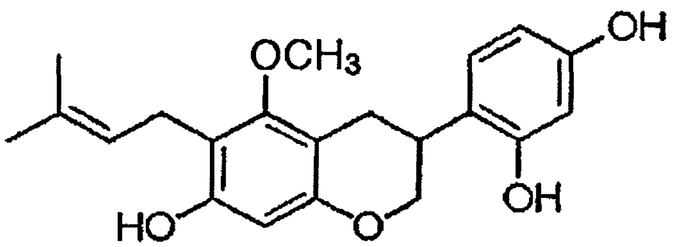

[Formula 5]

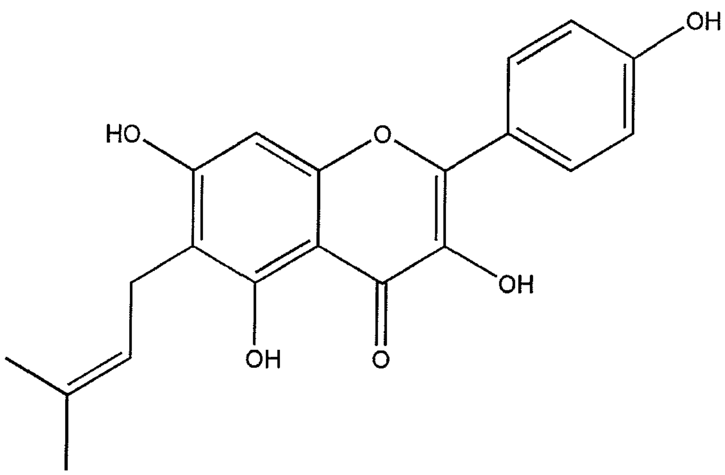

The composition of the present invention exhibits not only virucidal effects against rotavirus, but also the effect of inhibiting the cytopathic effect of rotavirus. Thus, it can be effectively used for the prevention or treatment of rotavirus infection.

As used herein, the term "prevention" refers to all actions that inhibit or delay rotavirus infection by administration of compositions.

As used herein, the term "treatment" refers to all actions that restore or beneficially change the disease caused by rotavirus infection by administering compositions.

The rotavirus may be a human, porcine, bovine or goat rotavirus. In addition, the rotavirus can cause diseases, including enteritis, diarrhea, a cold, a sore throat, bronchitis and pneumonia.

Generally, licorice (*Glycyrrhiza uralensis*) is a perennial plant belonging to the family *Fabaceae,* and grows naturally or is cultivated in northern China, Siberia, southern Italy, Manchuria, Mongol regions, etc. Licorice is used as a poison-reducing component in all Chinese herbal formulations and is used as an antitussive (Rauchensteiner F. et al., JPharmBiomedAnal., 38(4), pp.594-600, 2005). In Europe, licorice is used as a sweetener in tobacco, gum and candies. Recent study results indicated that licorice reduces oxidative damage to kidney cells (Yokozawa T. et al., Free Radic Res., 39(2), pp.203-211, 2005) and protects liver cells from damage by cadmium (Kim SC.et al., Toxicology., 197 (3), pp.239-251, 2004).

Licorice that is used in the present invention may be commercially available or be collected or cultivated in nature.

The licorice extract can be prepared by using any conventional method known in the art, such as an ultrasonic extraction method, a filtration method or a reflux extraction method.

Preferably, the licorice extract can be obtained by washing licorice to remove impurities, followed by drying and crushing the dried licorice, and extracting the crushed licorice with water, a $C_1$-$C_4$ alcohol or a mixed solvent thereof. More preferably, the licorice extract may be prepared by extracting the crushed licorice with a $C_1$-$C_4$ alcohol. Most preferably, the licorice extract may be prepared by extracting the crushed licorice with methanol or ethanol. Herein, the extraction solvent is used in an amount 2-20 times the dry weight of licorice. For example, the licorice extract can be obtained by finely cutting dried licorice, placing it in an extractor followed by addition of a $C_1$-$C_4$ lower alcohol or a mixed solvent of the lower alcohols, preferably methanol or ethanol, allowing the solution to stand at room temperature for a specific period of time, and then filtering the solution, thereby obtaining an alcohol extract of licorice. Herein, the extraction is performed by allowing the solution to stand at room temperature for 1 week, after which the extract may be concentrated or freeze-dried accordingly.

Meanwhile, fractions of the licorice extract can be obtained by fractionation of the licorice extract. More specifically, the fractions of the licorice extract can be obtained by suspending the licorice extract in water and fractionating the suspension into hexane and ethyl acetate, thereby obtaining three fractions i.e. a hexane fraction, an ethyl acetate fraction and an aqueous fraction.

Furthermore, the present invention is directed to a pharmaceutical composition for preventing or treating rotavirus infection, comprising one or more of the compounds represented by formulae 1 to 5 as an active ingredient,.

The inventive compounds represented by formulae 1 to 5 are typical active components present in licorice, and isolation of these compounds can be performed in the following manner.

First, licorice is extracted with water, a $C_1$-$C_4$ alcohol or a mixed solvent thereof to obtain a licorice extract (step 1). The licorice that is used in the present invention may be cultivated or commercially available and is washed and dried prior to extraction. The alcohol used in the extraction process may be a lower alcohol, such as methanol, ethanol, propanol or butanol. Preferably, the alcohol may be methanol or ethanol.

In step 1 of obtaining the licorice extract, the licorice is dried in the shade, cut finely and powdered in order to increase the efficiency of extraction. Then, the powder is placed in an extractor, and a suitable amount of alcohol is added thereto. Then, the solution is allowed to stand at room temperature for 5 days, and filtered using filter paper. This extraction process can be repeated several times, and the extract can be concentrated or freeze-dried subsequently.

The licorice extract obtained in step 1 is suspended in water and fractionated into hexane and ethyl acetate to obtain licorice extract fractions (step 2). Herein, a conventional fractional extraction method may be used. Preferably, a separating funnel may be used for extraction. The licorice extract fractions can be obtained as a hexane fraction, an ethyl acetate fraction, a butanol fraction, and an aqueous fraction.

Further, the licorice extract fractions obtained in step 2 can be further separated and purified by silica gel chromatography, thereby obtaining the compounds of formulae 1 to 5 (step 3). For silica gel chromatography to isolate the compounds, the mobile phase used in the chromatography is preferably n-hexane, n-hexane acetate, a mixed solvent of chloroform and acetone, and methanol. In additional chromatography, a mixed solvent of n-hexane and acetone may be used. Herein, the volume ratio between n-hexane and ethyl acetate in the mixed solvent is preferably 50:1-1:5, and the volume ratio between chloroform and acetone in the mixed solvent is preferably 150:1-1:4. The chromatography can be carried out once or several times until single compounds are obtained, and if necessary, the obtained compounds may be further concentrated or recrystallized.

In addition, each of the compounds represented by formulae 1 to 5 may be used in the form of a pharmaceutically acceptable salt. Useful are acid addition salts with pharmaceutically acceptable free acids. Acid addition salts may be obtained with inorganic acids such as hydrochloric acid, nitric acid, phosphoric acid, sulfuric acid, hydrobromic acid, hydriodic acid, nitric acid, phosphorous acid, etc., or non-toxic organic acids such as aliphatic mono- and dicarboxylate, phenyl-substituted alkanoate, hydroxy alkanoate and alkanedioate, aromatic acids, aliphatic and aromatic sulfonic acids. Examples of the pharmaceutically acceptable non-toxic salts include sulfate, pyrosulfate, bisulfate, sulfite, bisulfate,

nitrate, phosphate, monohydrogen phosphate, dihydrogen phosphate, metaphosphate, pyrophosphate chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, butyn-1,4-dioate, hexan-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutyrate, citrate, lactate, β-hydroxybutyrate, glycolate, malate, tartrate, methanesulfonate, propanesulfonate, naphthalene-1-sulfonate, naphthalene-2-sulfonate, and mandelate.

The acid addition salts according to the present invention may be prepared by conventional methods, for example by dissolving the compound of formula 1 in excess of an acid addition salt and precipitating the resulting salt in water-miscible organic solvent, for example, methanol, ethanol, acetone or acetonitrile.

In addition, pharmaceutically acceptable metal salts of the compounds represented by formula 1 to 5 can be prepared using bases. Alkali metals or alkali earth metals may be obtained, for example by dissolving the compound in excess of an alkali metal hydroxide or an alkali earth metal hydroxide solution, filtering the non-soluble compound salts, evaporating the filtrate, and drying it. Herein, suitable pharmaceutically acceptable metal salts include sodium, potassium or calcium salts. In addition, the corresponding silver salts are obtained by reacting alkali metals or alkali earth metals with a suitable silver salt (e.g., silver nitrate).

The pharmaceutical composition of the present invention may include pharmaceutically acceptable carriers. The composition may be in the form of various oral or parenteral formulations. The composition is formulated using conventional diluents or excipients, including fillers, extenders, binders, wetting agents, disintegrants, and surfactants.

Solid formulations for oral administration include tablets, pills, powders, granules, capsules, etc. These solid formulations may be prepared by mixing at least one compound with one or more excipients, for example, starch, calcium carbonate, sucrose, lactose, gelatin, etc. In addition to simple excipients, lubricants such as magnesium stearate and talc may be used.

In addition, liquid formulations for oral administration include a suspension, a solution, an emulsion and a syrup, etc. In addition to water commonly used as a simple diluent and liquid paraffin, various excipients, for example, wetting agents, sweetening agents, flavors, preservatives, etc. may be included. Formulations for parenteral administration include sterilized aqueous solutions, non-aqueous solvents, suspending agents, emulsions, freeze-drying agents, suppositories, etc. Propylene glycol, polyethylene glycol, vegetable oils such as olive oil, injectable esters such as ethyl oleate, etc. may be used as non-aqueous solutions and suspending agents. Suppositories may include witepsol, macrogol, tween 61, cacao butter, laurin butter, glycerinated gelatin, etc.

The pharmaceutical composition may have any one formulation selected from the group consisting of a tablet, a pill, powder, granules, a capsule, a suspension, a solution, an emulsion, a syrup, a sterilized aqueous solution, a non-aqueous solution, a suspension, an emulsion, a lyophilized formulation, and a suppository.

The pharmaceutical composition of the present invention may be administered in a pharmaceutically effective amount. As used herein, the term "pharmaceutically effective amount" refers to an amount sufficient to treat diseases, at a reasonable benefit/risk ratio applicable to any medical treatment. The effective dosage level of the composition may be determined depending on the subject's type, the disease severity, the subject's age and sex, the type of infected virus, the activity of the drug, sensitivity to the drug, the time of administration, the route of administration, excretion rate, the duration of treatment, drugs used in combination with the composition, and other factors known in the medical field. The pharmaceutical composition of the present invention may be administered alone or in combination with other therapeutic agents, and may be administered sequentially or simultaneously with conventional therapeutic agents. The composition can be administered in a single or multiple dosage form. It is important to administer the composition in the minimum amount that can exhibit the maximum effect without causing side effects, in view of all the above-described factors, this amount can be easily determined by a person skilled in the art.

In order to prevent or treat rotavirus infection, the pharmaceutical composition of the present invention may be used alone or in combination with surgery, hormonal therapy, drug therapy and a biological reaction regulator.

In another aspect, the present invention provides a method for preventing or treating rotavirus infection disease, the method comprising a step of administering a pharmaceutically effective amount of said pharmaceutical composition for preventing or treating rotavirus infection to a subject having or being at risk of developing rotavirus infection disease.

In still another aspect, the present invention provides the use of one or more of a licorice extract, fractions thereof and the compounds represented by formulae 1 to 5 in the preparation of a medicament for preventing or treating rotavirus infection disease.

The rotavirus may be human, porcine, bovine or goat rotavirus. In addition, the rotavirus can cause diseases, including enteritis, diarrhea, a cold, a sore throat, bronchitis and pneumonia.

As used herein, the term "subject" means all animals, including humans, who have already been infected or can be infected with rotavirus. The above disease can be effectively prevented and treated by administering the pharmaceutical composition of the present invention to the subject. For example, the pharmaceutical composition of the present invention can be used to treat not only humans infected with various human rotavirus subtypes or mutants, but

also pigs, cattle or goats infected with various rotavirus subtypes or mutants. The pharmaceutical composition of the present invention may be administered in combination with conventional agents for treating rotavirus infection disease.

The pharmaceutical composition of the present invention may be administered by any general route, as long as it can reach a target tissue. Specifically, the pharmaceutical composition of the present invention may be administered intraperitoneally, intravenously, intramuscularly, subcutaneously, intradermally, orally, locally, intranasally, intrapulmonarily or intrarectally, but is not limited thereto. In addition, the pharmaceutical composition of the present invention may be administered using any system capable of delivering the active ingredient to target cells.

In still another aspect, the present invention provides a method for inhibiting the activity of rotavirus, the method comprising a step of contacting the pharmaceutical composition with rotavirus. Herein, the contacting may be carried out by a conventional method, and the rotavirus may be included in living organisms, tissues or cell cultures, or biological samples (such as blood, serum, urine, cerebrospinal fluid, tear, sputum, saliva, tissue samples, etc.) and may generally include pathogenic organisms such as viruses. These samples may be included in any medium containing water or an organic solvent/water mixture.

In addition, after the pharmaceutical composition has been administered, observation may be performed by any methods, including direct or indirect methods of diagnosing anti-rotavirus activity. Quantitative, qualitative or semiquantitative methods of diagnosing the activity of inhibiting rotavirus infection may all be used, and other methods of observing the physiological characteristics of living organisms may also be used.

In yet another aspect, the present invention provides a food composition for preventing or ameliorating rotavirus infection, which contains, as an active ingredient, one or more of a licorice extract, fractions thereof and the compounds represented by formulae 1 to 5. Specifically, the licorice extract, fractions thereof or compounds separated therefrom of the present invention may be added to a food composition for preventing or treating rotavirus infection.

If the licorice extract, fractions thereof or compounds separated therefrom are used as a food additive, they may be used alone or in combination with other foods or food components and may be used appropriately according to conventional methods. The amount of active ingredient added may be suitably determined depending upon the intended use (prophylactic, health promotion or therapeutic treatment). Generally, when a food or a beverage is produced, the composition of the present invention is added in an amount of 0.01-10 wt%, preferably 0.05-1 wt%, based on the total weight of said food or beverage. However, when long-term intake is intended for the purpose of health and hygiene or for health control, the amount of the composition of the present invention may be smaller than the lower limit of the above-specified range.

There is no particular limit to the kind of food composition. Examples of foods to which the composition of the present invention can be added include meats, sausages, bread, chocolate, candies, snack, confectionery, pizza, noodles, gums, dairy products including ice cream, various soups, beverages, teas, drinks, alcoholic beverages and multivitamin preparations. The health foods include all health foods in a conventional sense.

The health beverage compositions of the present invention may additionally contain various sweetening agents or natural carbohydrates as in conventional beverages. The natural carbohydrates include monosaccharides, such as glucose and fructose, disaccharides, such as maltose and sucrose, polysaccharides, such as dextrin and cyclodextrin, and sugar alcohols, such as xylitol, sorbitol, and erythritol. Sweeteners include natural sweeteners such as thaumatin and stevia extracts, and synthetic sweeteners, such as saccharin and aspartame. The natural carbohydrates are used in an amount of about 0.01-0.04 g, and preferably about 0.02-0.03 g, based on 100 ml of the composition of the present invention.

In addition, the food composition of the present invention may contain various nutrients, vitamins, electrolytes, flavoring agents, colorants, pectic acid or its salt, alginic acid or its salt, organic acids, protective colloidal tackifiers, pH adjusters, stabilizers, preservatives, glycerin, alcohol, carbonating agents used in carbonated drinks, etc. Additionally, the food composition of the present invention may contain fruit flesh for the preparation of natural fruit juices, fruit juice beverages and vegetable juices. These components may be used alone or in combination. Although not critical, these additives are used in an amount of 0.01-0.1 parts by weight based on 100 parts by weight of the food composition of the present invention. These components may be used alone or in combination.

In yet another aspect, the present invention provides a virucidal agent against rotavirus, comprising one or more of a licorice extract, fractions thereof or compounds represented by formulae 1 to 5 as an active ingredient. Specifically, the licorice extract, fractions thereof or the compounds separated therefrom of the present invention may be added to a virucidal agent for preventing or treating rotavirus infection.

If the inventive licorice extract, fractions thereof or compounds separated therefrom are used as virucidal agents, the licorice extract, fractions thereof or the compounds separated therefrom may be used alone or in combination with other virucidal agents and can be appropriately used according to conventional methods. The amount of active ingredient added can be determined according to the intended use.

Preferably, the virucidal agent can be prepared in the form of a disinfectant, shower foam, a mouth wash, a wet tissue, a detergent soap, a hand wash, a filler for humidifiers, a facial mask, an ointment or a filler for filters.

Mode for Invention

Hereinafter, the present invention will be described in further detail with reference to examples and test examples. It is to be understood, however, that these examples are for illustrative purposes only and are not intended to limit the scope of the present invention.

Example 1: Preparation of licorice extract

2 kg of licorice roots, obtained in Jeongeup, Jeollabuk-do, South Korea, were added to 10 L of 100% ethanol (EtOH) and allowed to stand at room temperature for 3 days. Further, the solution was filtered through filter paper and concentrated, thereby obtaining an ethanol extract of licorice (134 g).

**Example 2: Separation and purification of fractions and polyphenolic compounds from licorice extract**

134 g of the brown powder obtained as the ethanol extract of licorice in Example 1 was suspended in 1 L of water. The suspension was placed in a separating funnel and fractionally extracted with n-hexane, chloroform and ethyl acetate, thereby obtaining an n-hexane soluble extract (1.3 g), a chloroform soluble extract (45 g), ethyl acetate soluble extract (35 g), and a water soluble extract.

20 g of the obtained chloroform soluble extract was further separated into 10 fractions (Fr.1-10) by silica gel column chromatography (500 g silica gel; 70 -230 mesh) using 100% chloroform, acetone and a mixed solvent thereof (40: 1- 1: 1) as a mobile phase. Among these fractions, the third fraction (Fr.3, 3 g) was further subjected to silica gel column chromatography (7 g silica gel; 230-400 mesh) using a mixed solvent of n-hexane: ethyl acetate (40: 1-2: 1 (v/v)), thereby obtaining 30 fractions (Fr.3-1-30). Among these fractions, the Fr.3-18-20 fractions (0.11 g) were subjected to preparative TLC using a mixed solvent of n-hexane: ethyl acetate = 4 : 1 (v/v), thereby obtaining compound 5 (13 mg).

In addition, 17 g of the ethyl acetate soluble extract was separated into 14 fractions (Fr.1~14) by silica gel column chromatography (500 g silica gel; 70-230 mesh) using 100% n-hexane, ethyl acetate and a mixed solvent thereof (60: 1-1: 4) as a mobile phase. Among these fractions, the second fraction (Fr.2; 1.5 g) was further subjected to silica gel column chromatography (30 g silica gel; 230-400 mesh) using a mixed solvent of n-hexane: ethyl acetate (50: 1-2: 1 (v/v)) as a mobile phase, thereby obtaining 10 fractions (Fr.3-1~10). Among these fractions, the Fr.3-4 fraction (0.21 g) was subjected to column chromatography using Sephadex-LH20, thereby obtaining compound 4 (32 mg). Also, the Fr.3-8 fraction was subjected to the same column chromatography, thereby obtaining compound 1 (13 mg).

In addition, the tenth fraction (Fr.10; 1.2 g) of the chloroform soluble extract was subjected to silica gel column chromatography (40 g silica gel; 230-400 mesh) using n-hexane: ethyl acetate (30: 1-1: 2) as a mobile phase solvent, thereby obtaining 20 fractions (Fr.10-1~20). Among these fractions, the Fr.10-12~15 fractions were further subjected to silica gel column chromatography, thereby obtaining compound 1 (14 mg) and compound 2 (54 mg).

Furthermore, the fractions Fr.10-15~20 of the chloroform soluble extract were subjected to Sephadex-LH20 chromatography and then silica gel column chromatography, thereby obtaining compound 3 (20 mg).

**Example 3: Structural analysis of compounds**

The molecular weights and molecular formulae of the compounds obtained in Example 2 were determined using a VG high-resolution GC/MS spectrometer (Election Ionization MS, Autospec-Ultima). In addition, using the spectrometry data of $^1$H NMR and $^{13}$C NMR by nuclear magnetic resonance (NMR) analysis (Bruker AM 300, 500), the molecular structures of the compounds were determined.

The obtained instrumental analysis results were analyzed comparatively with those of the literature. As a result, it was found that the compounds are isoliquiritigenin represented by the formula 1, 2-methoxyisoliquiritigenin represented by the formula 2, licocoumarone represented by the formula 3, glyasperin C represented by the formula 4, and licoflavonol represented by the formula 5, respectively (J. Agric. Food Chem. 7408-7414, 2005; Phytochemistry, 287-293, 1998; Chem. Nat. Comp., 389, 1972; Chem. Pharm. Bull., 1286-1292, 2000). The specific results of the analysis are as follows:

<u>Compound 1: Isoliquiritigenin</u>

[Formula 1]

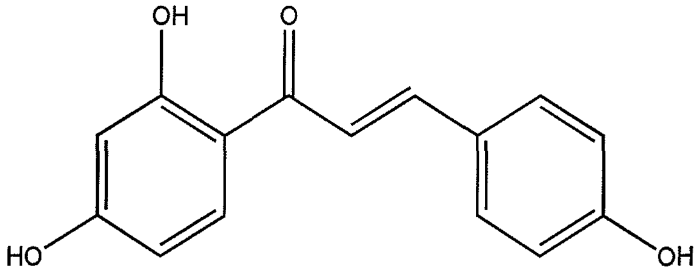

1) Appearance: yellow solid (m.p. 206-210 °C)
2) Molecular weight: 256
3) Molecular formula: $C_{15}H_{12}O_4$
4) $^1$H-NMR (methanol-$d_3$, 500 MHz) δ 6.28, 6.41, 6.83, 7.59, 7.61, 7.78, 7.95. $^{13}$C-NMR (methanol-$d_3$, 125 MHz) δ 193.7, 167.7, 166.6, 161.7, 145.8, 133.5, 131.9, 128.0, 118.5, 117.0, 114.8, 109.3, 103.9.

Compound 2 2-methoxyisoliquiritigenin

[Formula 2]

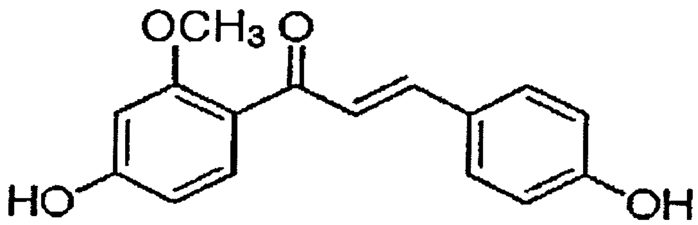

1) Appearance: yellow solid (m.p. 89-92 °C)
2) Molecular weight: 270
3) Molecular formula: $C_{16}H_{14}O_4$
4) $^1$H-NMR (methanol-$d_3$, 500 MHz) δ 3.78, 6.37, 6.78, 7.49, 7.86, 7.87. $^{13}$C-NMR (methanol-$d_3$, 125 MHz) δ 56.5, 100.4, 109.7, 116.76, 117.2, 119.9, 131.9, 132.1, 132.5, 141.7, 162.6, 163.6, 164.0, 192.1.

Compound 3 licocoumarone

[Formula 3]

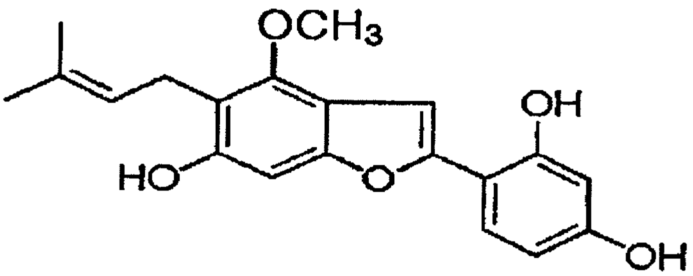

1) Appearance: yellow solid (m.p. 183-185 °C)
2) Molecular weight: 340

3) Molecular formula: $C_{20}H_{20}O_5$

4) $^1$H-NMR (acetone-$d_6$, 300 MHz) δ 1.65, 1.79, 3.42, 5.27, 6.50, 6.57, 6.78, 7.31, 7.69. $^{13}$C-NMR (acetone-$d_6$, 75 MHz) δ 158.2, 155.2, 153.8, 153.3, 151.1, 150.6, 129.5, 127.1, 124.1, 114.7, 113.6, 110.1, 107.4, 103.1, 101.0, 92.4, 59.5, 25.0, 22.5, 17.0.

Compound 4: glyasperin C

[Formula 4]

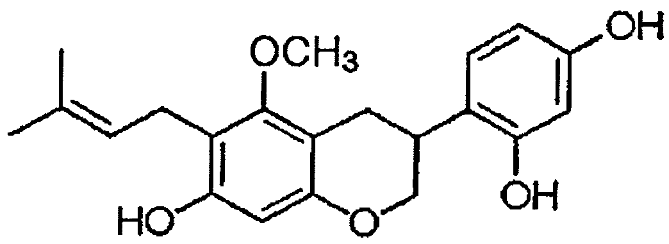

1) Appearance: white powder

2) Molecular weight: 356

3) Molecular weight : $C_{21}H_{24}O_5$

4) $^1$H-NMR (methanol-$d_4$, 300 MHz) δ 1.50, 1.59, 2.60, 2.71, 3.08, 3.19, 3.51, 3.76, 4.01, 5.04, 5.93, 6.10, 6.16, 6.72. $^{13}$C-NMR (methanol-$d_4$, 75 MHz) δ 18.3, 24.0, 26.3, 27.2, 33.2, 61.3, 71.4, 100.4, 103.9, 108.5, 109.0, 115.5, 120.7, 126.0, 129.2, 131.2, 155.2, 156.2, 157.6, 158.4, 158.8.

Compound 5: Licoflavonol

[Formula 5]

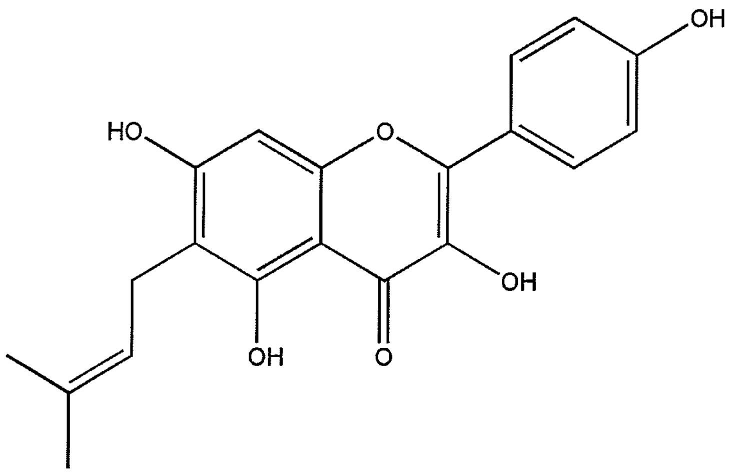

1) Appearance: yellow powder

2) Molecular weight: 354 (m.p. 120-130 °C)

3) Molecular formula : $C_{20}H_{18}O_6$

4) $^1$H-NMR (acetone-$d_6$, 300 MHz) δ 1.66, 1.78, 3.31, 5.24, 6.42, 6.89, 8.07. $^{13}$C-NMR (acetone-$d_6$, 75 MHz) δ 18.0, 22.3, 26.1, 93.7, 104.4, 112.3, 116.4, 123.7, 124.0, 130.7, 132.0, 137.2, 147.8, 156.3, 159.2, 160.6, 163.6, 177.4.

**Test Example 1: Measurement of inhibitory effects of licorice extract, fractions thereof and compounds separated therefrom on rotavirus**

In order to the CPE (cytopathic effect) reduction effects and virucidal effects of the compounds separated from licorice on bovine rotavirus NCDV (G6P[1]) and porcine rotavirus KJ205 (G5P[7]), the following in vitro experiment was performed using the fetal rhesus monkey kidney cell line TF-104.

First, TF-104 cells were added to a 96-well microplate at a density of 4 X $10^4$ cells/well and cultured with alpha-MEM medium (penicillin 100 units, streptomycin 100 mg, 5% FBS). When the TF-104 cells formed a monolayer, the cells were washed twice with alpha-MEM containing only an antibiotic. Each of NCDV and KJ205 was diluted to an MOI of 0.01 and placed in EP tubes. Each of the licorice extract and the polyphenolic compounds, diluted with dimethylsulfoxide (DMSO), was placed in each of the tubes at varying concentrations and then allowed to react at 4 °C for 1 hour. After 1 hour, the reaction material was inoculated into the washed TF-104 cells in such a manner that one sample concentration was inoculated into 3 wells (hereinafter referred to as "sample-treated groups"). Meanwhile, a non-infected + non-administered control (cell group not infected with NCDV or KJ205 and not treated with the compound) and an infected + non-administered control (virus control; a cell group infected with NCDV or KJ205, but not treated with the compound) were allowed to react under the same conditions for 1 hour, and then inoculated into the TF-104 cells, which were then incubated at 37 °C for 1 hours. After 1 hour, the medium was completely removed from the plate, which was then washed once with PBS. Then, 100 mL of alpha-MEM medium containing 1 mg/mL of trypsin and antibiotic were dispensed into each well of the plate and incubated at 37 °C for 5-6 days. In the case of the infected + non-administered control group, incubation was performed for 5-6 days until the cytopathic effect (CPE) of the virus clearly appeared. The state of the cells was observed with an inverted microscope every day. After 5-6 days of the incubation, in order to examine the viability of the cells, 1 mL of a neutral red solution (Sigma, UK) was added to each well of the place and allowed to react at 37 °C for 2 hours. Then, the medium was removed, 100 mL of a destaining solution (1 % glacial acetic acid, 49 % DDW, 50 % EtOH) was added to each well, and after 15 minutes, the absorbance at 540 nm was measured. The virucidal effects (inhibition %) of the inventive compounds separated from licorice compared to the non-infected + non-administered control group and the infected + non-administered control group were calculated using the following equation 1, and the results of the calculation are shown in Table 1 below.

[Equation 1]

Inhibition % = (O.D) value of sample-treated group - (O.D) value of infected + non-administered control / (O.D) value of non-infected + non-administered control - (O.D) value of infected + non-administered control x 100

In the above equation, the O.D value means the absorbance measured at 540 nm.

Meanwhile, in order to examine the inhibitory effects of the compounds separated from licorice on the CPE of bovine rotavirus NCDV (G6P[1]) and porcine rotavirus KJ205 (G5P[7]), TF-10 cells were washed twice with alpha-MEM containing only an antibiotic, after which the viruses were inoculated into the TF-104 cells and incubated at 37 °C for 1 hour. After 1 hour, the inoculated virus liquids were completely removed, the virus-inoculated TF-104 cells were treated with varying concentrations of the licorice extract and the polyphenolic compounds. Further, the cytopathic effect inhibition (inhibition %) of the compounds separated from licorice were calculated using equation 1 above in the same manner as described above, and the results of the calculation are shown in Table 1 below.

[Table 1]

| Sample | Virucidal effect | | | | | |
|---|---|---|---|---|---|---|
| | NCDV (G6P[1] | | | KJ205 (G5P[7]) | | |
| | $CC_{50}$[1)] (μM) | $EC_{50}$[2)] (μM) | S.I.[3)] | $CC_{50}$ (μM) | $EC_{50}$ (μM) | S.I. |
| Ethanol extract | 368.6 | - | - | 368.6 | 22.4 | 16.4 |
| Compound 3 | - | - | - | 59.6 | 26.1 | 2.3 |

(continued)

| | $CC_{50}$[1)] (μM) | $EC_{50}$[2)] (μM) | S.I.[3)] | $CC_{50}$ (μM) | $EC_{50}$ (μM) | S.I. |
|---|---|---|---|---|---|---|
| Compound 4 | 39.1 | 26.2 | 1.5 | 39.1 | 18.7 | 2.1 |
| Sample | CPE inhibition effect | | | | | |
| | NCDV (G6P[1] | | | KJ205 (G5P[7]) | | |
| | $CC_{50}$ (μM) | $EC_{50}$ (μM) | S.I. | $CC_{50}$ (μM) | $EC_{50}$ (μM) | S.I. |
| Ethanol extract | 368.6 | 118.7 | 3.1 | 368.6 | 27.4 | 13.5 |
| Compound 1 | - | - | - | 137.9 | 35.3 | 3.9 |
| Compound 2 | - | - | - | 279.9 | 24.0 | 11.7 |
| Compound 3 | - | - | - | 59.6 | 26.1 | 2.3 |
| Note: 1) 50% concentration from cytotoxic concentration; 2) 50% concentration from inhibition concentration; 3) selective index, $CC_{50}/EC_{50}$ | | | | | | |

As can be seen in Table 1 above, the inventive licorice extract and the compounds separated therefrom showed excellent inhibitory activities against various rotavirus strains. The licorice extract and compounds 3 and 4 separated therefrom showed a high selective index of 1.5-16.4, suggesting that they have excellent virucidal effects. Compound 4 showed selective virucidal effects against porcine rotavirus, but compound 3 showed good inhibitory activities against bovine and porcine rotaviruses. Such results indicate that the licorice extract and the compounds separated therefrom have inhibitory activities against various rotaviruses.

In addition, as can be seen in Table 1 above, the licorice extract showed a selective index of 3.1 in the bovine rotavirus and a selective index of 13.5 in the porcine rotavirus, it has inhibitory effects on the cytopathic effects of various rotaviruses. Moreover, it could be seen that compounds 1 to 3 had excellent inhibitory effects on the cytopathic effect of porcine rotavirus. In particular, compound 2 had a $CC_{50}$ of 279.9 μM, an $EC_{50}$ of 24.0 μM and a selective index of 11.7, suggesting that it has low toxicity and a very high virucidal effect.

As described above, the composition of the present invention shows excellent virucidal effects, which act directly on virus before infection of cells with the virus, and excellent inhibitory effects on the cytopathic effect of virus (replication of virus after infection of cells with the virus). Thus, the composition of the present invention can be effectively used for the prevention and treatment of rotavirus infection disease.

**Test Example 2: Acute toxicity test for compounds 1 to 5**

In order to examine the acute toxicities of the compounds obtained in Example 2, the following test was carried out.

6 week-old SPF (specific pathogen-free) C57BL/6J mice were divided into 4 groups (3 males or 3 females per test group) and bred in an animal chamber at a temperature of 22 ± 3 °C and a humidity of 55 ± 10 % under a 12-hr light/12-hr dark cycle (12L/12D). The mice were acclimated for about 1 week before they were used in the test. Animal feed (for mice and rats; CJ CheilJedang, Seoul, Korea) and water were fed after sterilization, and the mice were allowed access to the feed and water ad libitum.

Specifically, compound 2 prepared in Example 2 was dissolved in 0.5% tween 80 at a concentration of 50 mg/mL, and then administered orally to the mouse groups in amounts of 0 mL/20 g, 0.04 mL/20 g (100 mg/kg), 0.2 mL/20 g (500 mg/kg) and 0.4 mL/20 g (1,000 mg/kg), respectively. The sample was administered orally once, and for 7 days after the administration, the observation of adverse effects or deaths was performed. Specifically, the change in general conditions and the presence or absence of dead animals was observed 1, 4 8 and 12 hours after administration on day 1 and one or more times in the morning and evening on days 2-7.

As a result, in the mice administered with the samples prepared in Example 2, no special clinical symptom appeared, and no dead mouse was observed. In addition, in the results of measurement of weight, blood examination, serum biochemical examination, autopsy and the like, no change in toxicity was observed.

Thus, it could be seen that the compounds of the present invention showed no change in toxicity up to 1,000 mg/kg in the mice and that the oral lethal dose ($LD_{50}$) thereof was 1,000 mg/kg or more, suggesting that these compounds are safe.

Hereinafter, examples of pharmaceutical formulations or health foods comprising a licorice extract, fractions thereof or compounds separated therefrom or salts thereof will be described.

**Preparation Example 1: Preparation of pharmaceutical formulations**

**1-1. Preparation of powder**

Licorice extract, fractions thereof, or compound separated therefrom or salts thereof: 2g
Lactose: 2 g
The above compounds were mixed and filled in an airtight pack to prepare a powder formulation.

**1-2. Preparation of tablet**

Licorice extract, fraction thereof, or compound separated therefrom or salts thereof: 100 mg
Corn starch: 100 mg
Lactose: 100 mg
Magnesium stearate: 2 mg
The above components were mixed and compressed into a tablet according to a conventional method.

**1-3. Preparation of capsule**

Licorice extract, fraction thereof, or compound separated therefrom or salt thereof: 100 mg
Corn starch: 100 mg
Lactose: 100 mg
Magnesium stearate: 2 mg
The above components were mixed and filled in a gelatin shell according to a conventional method, thereby preparing a capsule formulation.

**1-4. Preparation of injectable liquid formulation**

Licorice extract, fraction thereof, or compound separated therefrom or salt thereof: 10 μg/mL
Dilute hydrochloric acid BP: to a pH of 3.5
Sodium chloride BP for injection: up to 1 mL

A licorice extract, a fraction thereof, a compound separated therefrom or a salt thereof was dissolved in a suitable volume of injectable sodium chloride BP and was adjusted to a pH of 3.5 using dilute hydrochloric acid BP. Injectable sodium chloride BP was further added to achieve a desired volume, and the solution was sufficiently mixed. The mixture solution was then filled into a 5-mL type I ampoule made of transparent glass. The glass was melted to seal the ampoule, which was autoclaved at 120 °C for 15 min or more, thereby obtaining an injectable solution.

**Preparation Example 2: Preparation of health foods**

**2-1. Preparation of cooking condiment**

A cooking condiment for health promotion was prepared using 0.2-10 wt% of a licorice extract, a fraction thereof, or a compound separated therefrom or a salt thereof.

**2-2. Preparation of tomato ketchup and sauce**

0.2-1.0 wt% of a licorice extract, a fraction thereof, or a compound separated therefrom or a salt thereof was added to tomato ketchups or sauces to prepare tomato ketchups or sauces for health promotion.

**2-3. Preparation of flour food**

0.1-5.0 wt % of a licorice extract, a fraction thereof, or a compound separated therefrom or a salt thereof was mixed with flour, and the mixture was used to make breads, cakes, cookies, crackers, and noodles, thereby preparing foods for health promotion.

**2-4. Preparation of soup and gravy**

0.1-1.0 wt % of a licorice extract, a fraction thereof, or a compound separated therefrom or a salt thereof was added to soups and gravies to prepare processed meat products, soups for noodles, and gravies for health promotion.

### 2-5. Preparation of ground beef

10 wt % of a licorice extract, a fraction thereof, or a compound separated therefrom or a salt thereof was added to ground beef to prepare ground beef for health promotion.

### 2-6. Preparation of dairy product

0.1 to 1.0 wt % of a licorice extract, a fraction thereof, or a compound separated therefrom or a salt thereof was added to milk, and the milk was used to prepare various dairy foods, including butter and ice-cream.

### 2-7. Preparation of cereal powder

Unpolished rice, barley, glutinous rice, and adlay were pregelatinized by a known method, dried, roasted, and milled into 60-mesh powder using a mill.

Black soybean, black sesame, and perilla were steamed by a known method, dried, roasted, and milled into 60-mesh powder using a mill.

A licorice extract, a fraction thereof, or a compound separated therefrom or a salt thereof was evaporated under reduced pressure using a vacuum evaporator, sprayed, hot-air dried, and milled into dried powder having a particle size of 60 mesh using a mill.

The obtained cereals, seeds, and dried powder of the licorice extract, fraction, or compound separated therefrom or its salt were mixed at the following ratio.

Cereals (35 wt% of unpolished rice, 15 wt% of adlay, and 25 wt% of barley),
Seeds (7 wt% of perilla, 9 wt% of black soybean, and 7 wt% of black sesame),
1 wt% of the licorice extract, fraction, or compound separated therefrom or its salt,
0.5 wt% of Lingzhi mushroom.
0.5 wt% of Rehmanni.

### 2-8. Preparation of carbonated beverage

5-10 wt% of sugar, 0.05-0.3 wt% of citric acid, 0.005-0.02 wt% of caramel, and 0.01-1 wt% of vitamin C were mixed. 79-94 wt% of purified water was added thereto, yielding syrup. The syrup was sterilized at 85 to 98 °C for 20 to 180 seconds, and mixed with cooling water at a ratio of 1:4. Then, 0.5 to 0.82 volume % of carbonic acid gas was injected therein, thereby preparing a carbonated beverage comprising a licorice extract, a fraction thereof, or a compound separated therefrom or a salt thereof.

### 2-9. Preparation of health beverage

A licorice extract, a fraction thereof, or a compound separated therefrom or a salt thereof was uniformly mixed with additives including 0.5 wt% of liquid fructose, 2 wt% of oligosaccharide, 2 wt% of sugar, 0.5 wt% of table salt, and 94 wt% of water, then flash-pasteurized, and packed in a small packing container such as a vial, a PET bottle, and the like, to prepare health beverages.

### 2-10. Preparation of vegetable juice

0.5 g of a licorice extract, a fraction thereof, or a compound separated therefrom or a salt thereof was added to 1,000 mL of tomato or carrot juices to prepare vegetable juices for health promotion.

### 2-11. Preparation of fruit juice

0.1 g of a licorice extract, a fraction thereof, or a compound separated therefrom or a salt thereof was added to 1,000 ml of apple or grape juices to prepare fruit juices for health promotion.

## Claims

1. A pharmaceutical composition for the prevention or treatment of rotavirus infection, comprising one or more of a licorice extract, fractions thereof or compounds represented by the following formulae 1 to 5 as an active ingredient:

[Formula 1]

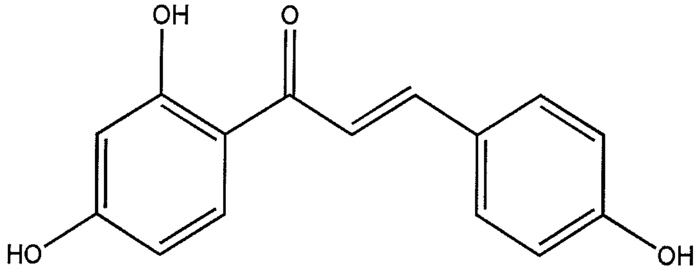

[Formula 2]

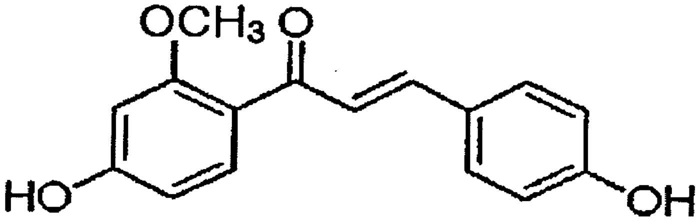

[Formula 3]

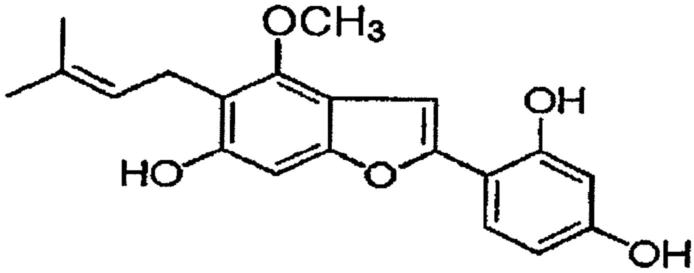

[Formula 4]

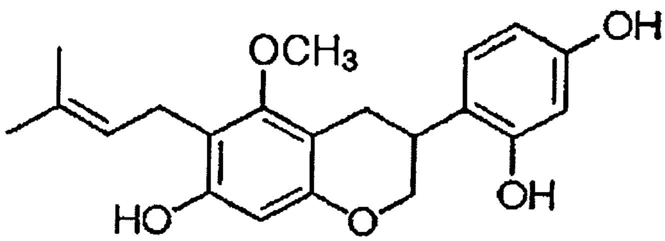

[Formula 5]

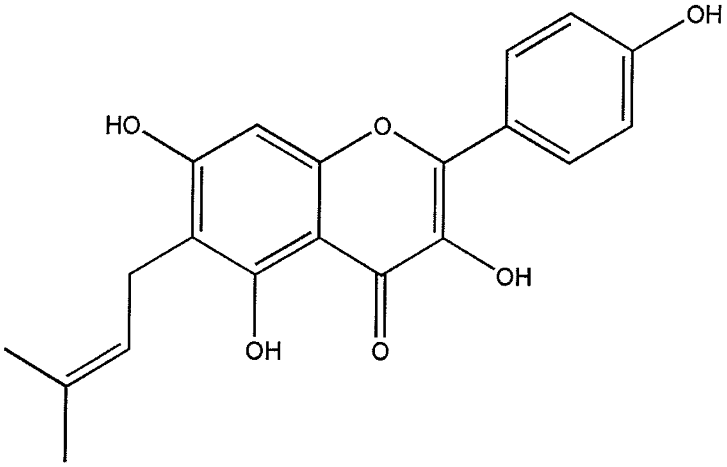

2. The pharmaceutical composition according to claim 1, wherein the extract is obtained by extraction with water, a $C_1$-$C_4$ alcohol or a mixed solvent thereof.

3. The pharmaceutical composition according to claim 1, wherein the fraction is a hexane fraction, an ethyl acetate fraction or an aqueous fraction.

4. The pharmaceutical composition according to claim 1, wherein the rotavirus is human, porcine, bovine or goat rotavirus.

5. The pharmaceutical composition according to claim 1, wherein the rotavirus causes enteritis, diarrhea, a cold, a sore throat, bronchitis or pneumonia.

6. The pharmaceutical composition according to claim 1, wherein the pharmaceutical composition is in the form selected form the group consisting of a tablet, a pill, powder, granules, a capsule, a suspension, a solution, an emulsion, a syrup, a sterilized aqueous solution, a non-aqueous solution, a suspension, an emulsion, a lyophilized formulation, and a suppository.

7. A food composition for the prevention or amelioration of rotavirus infection, comprising one or more of a licorice extract, fractions thereof or compounds represented by the following formulae 1 to 5 as an active ingredient:

[Formula 1]

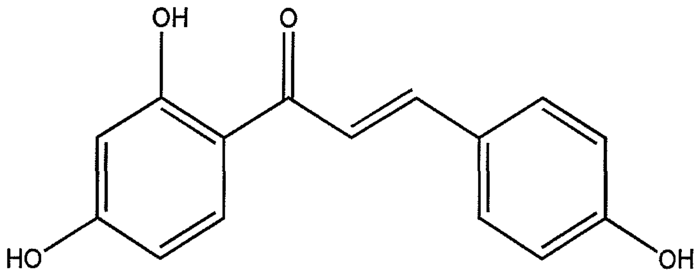

[Formula 2]

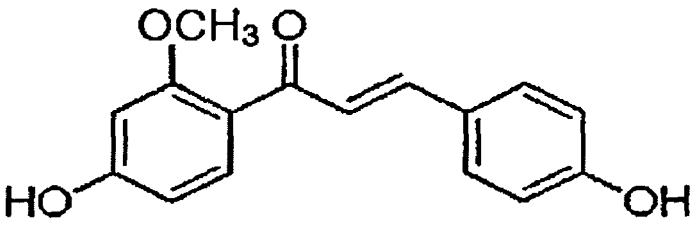

[Formula 3]

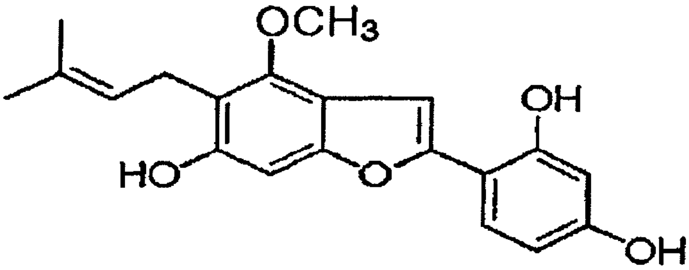

[Formula 4]

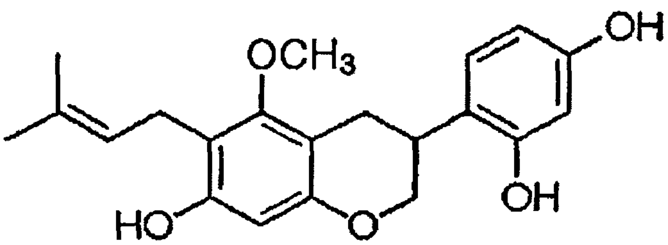

[Formula 5]

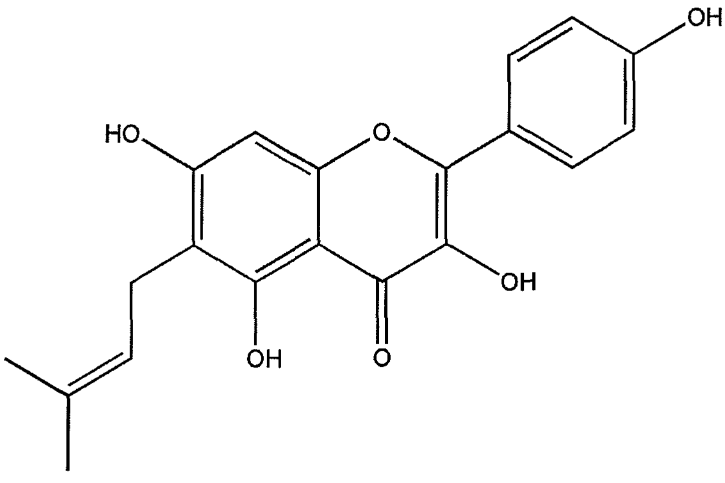

**8.** The food composition according to claim 7, wherein the food composition is any one selected from the group consisting of meats, sausages, bread, chocolate, candies, snack, confectionery, pizza, noodles, gum, ice cream, soups, beverages, teas, drinks, alcoholic beverages and multi-vitamin preparations.

**9.** A virucidal agent against rotavirus comprising one or more of a licorice extract, fractions thereof or compounds represented by the following formulae 1 to 5 as an active ingredient:

[Formula 1]

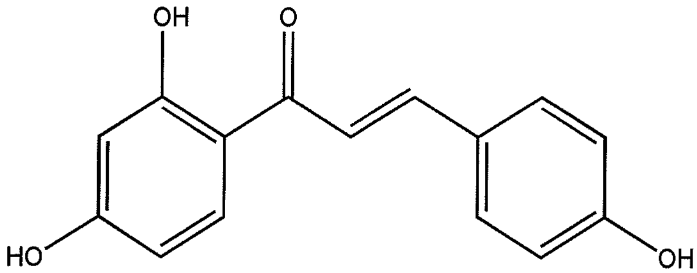

[Formula 2]

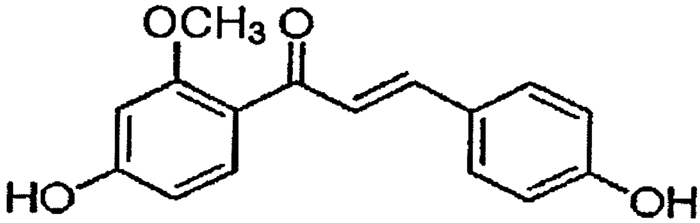

[Formula 3]

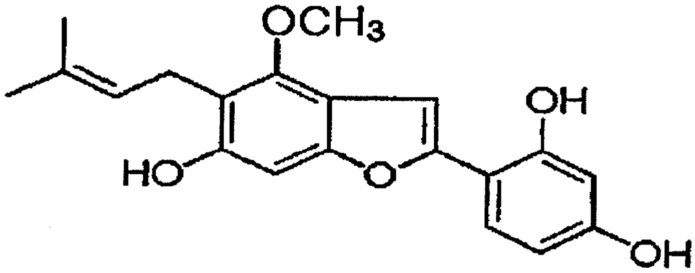

[Formula 4]

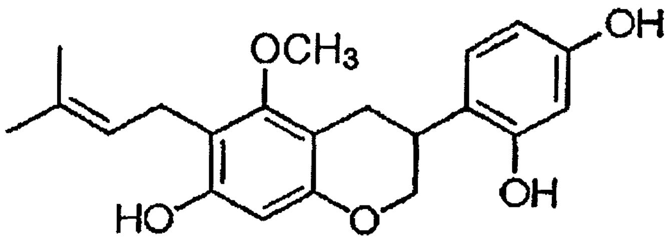

[Formula 5]

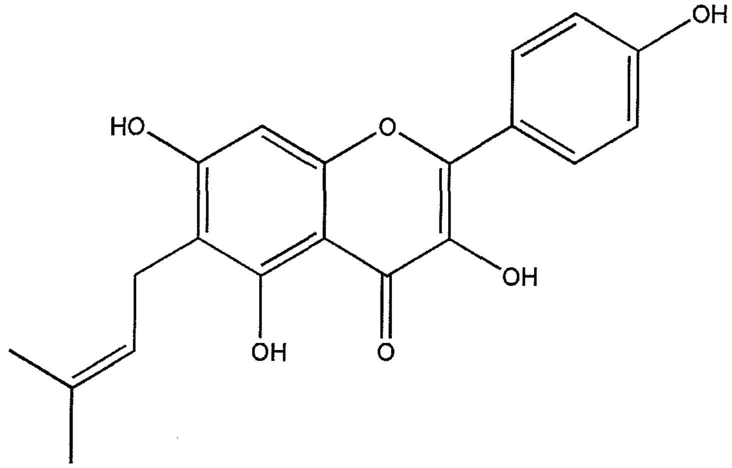

10. The virucidal agent according to claim 9, wherein the virucidal agent is prepared in the form of a disinfectant, shower foam, a mouth wash, a wet tissue, a detergent soap, a hand wash, a filler for humidifiers, a facial mask, an ointment or a filler for filters.

11. A method for preventing or treating rotavirus infection disease, comprising administering a pharmaceutical composition for preventing or treating rotavirus infection to a subject having or being at risk of developing rotavirus infection disease, wherein the composition comprises one or more of a licorice extract, fractions thereof or compounds represented by the following formulae 1 to 5 as an active ingredient:

[Formula 1]

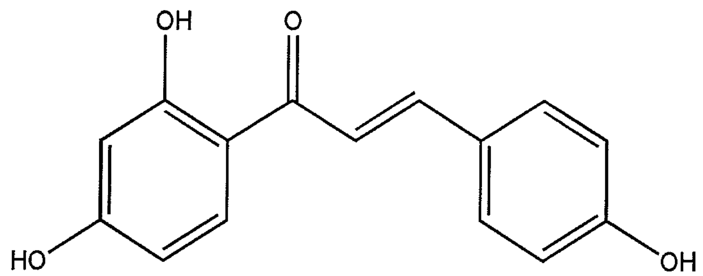

[Formula 2]

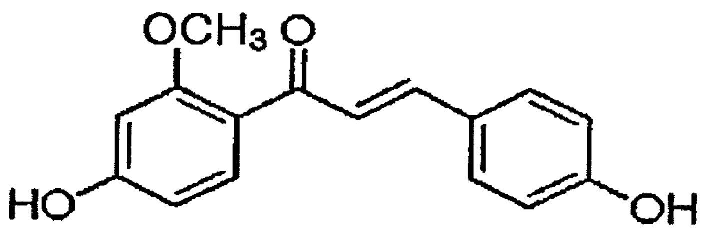

[Formula 3]

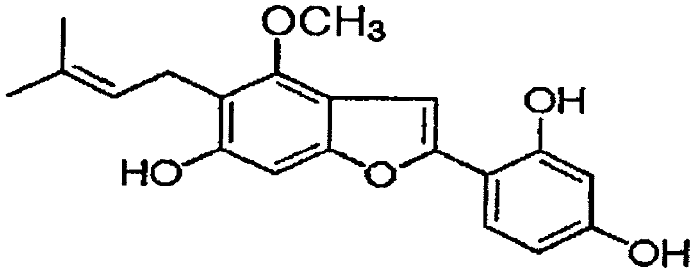

[Formula 4]

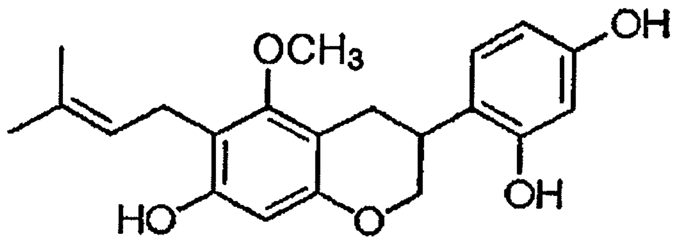

[Formula 5]

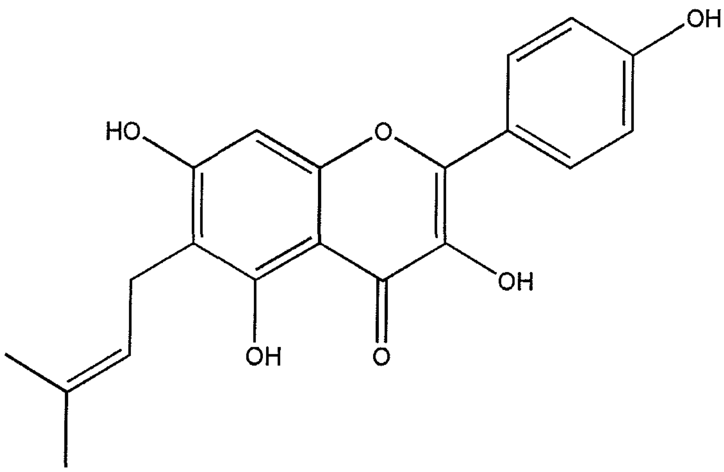

**12.** The method according to claim 11, wherein the rotavirus infection disease is any one or more selected from the group consisting of enteritis, diarrhea, a cold, a sore throat, bronchitis and pneumonia.

**13.** Use of one or more of a licorice extract, fractions thereof or compounds represented by the following formulae 1 to 5 in preparation of a medicament for treating rotavirus infection disease:

[Formula 1]

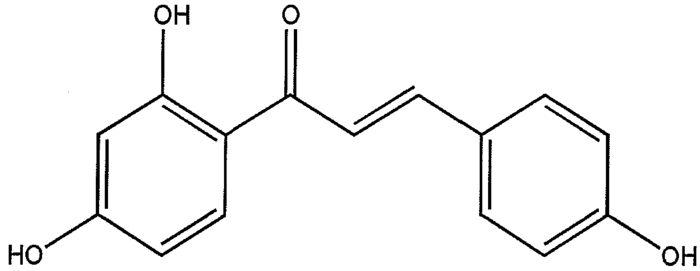

[Formula 2]

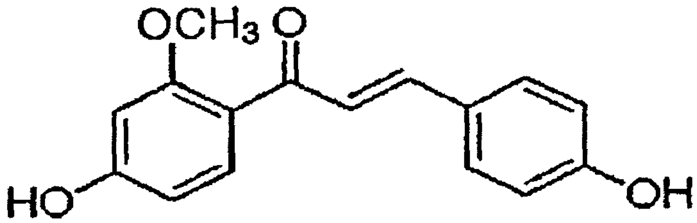

[Formula 3]

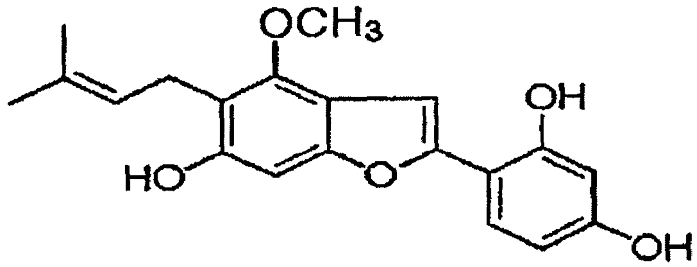

[Formula 4]

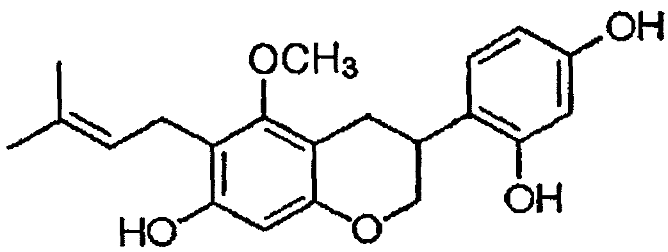

[Formula 5]

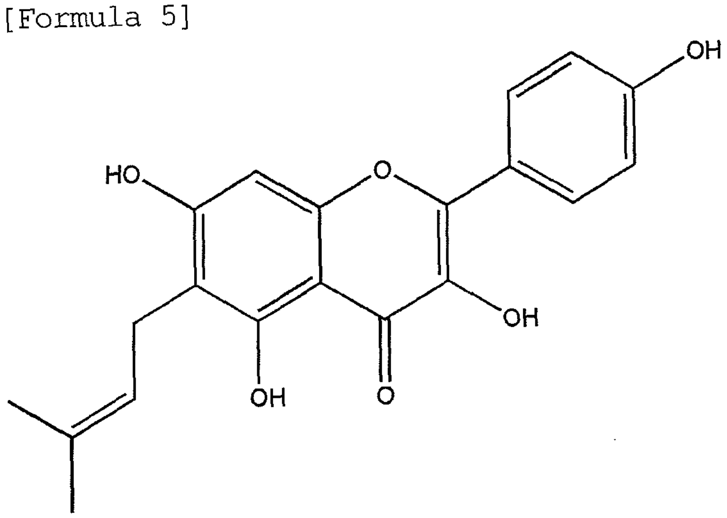

**14.** The use according to claim 13, wherein the rotavirus infection disease is any one or more selected from the group consisting of enteritis, diarrhea, a cold, a sore throat, bronchitis and pneumonia.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **ESTER, M. K.** *Rotaviruses and Their Replication in Fields Virology,* 2001, 1747-1785
- **CIARLET, M. ; ESTER, M. K.** Rotaviruses: basic biology, epidemiology and methodologies in encyclopedia of environmental microbiology. 2002, 2753-2773
- **TAKAHASHI, K et al.** *Antiviral Res.,* 2001, vol. 49, 15
- **RAUCHENSTEINER F. et al.** *JPharmBiomedAnal.,* 2005, vol. 38 (4), 594-600
- **YOKOZAWA T. et al.** *Free Radic Res.,* 2005, vol. 39 (2), 203-211
- **KIM SC. et al.** *Toxicology,* 2004, vol. 197 (3), 239-251
- *J. Agric. Food Chem.,* 2005, 7408-7414
- *Phytochemistry,* 1998, 287-293
- *Chem. Nat. Comp.,* 1972, 389
- *Chem. Pharm. Bull.,* 2000, 1286-1292